(19) Europäisches Patentamt
European Patent Office
Office européen des brevets

(11) Veröffentlichungsnummer : **0 297 442 B1**

(12) # EUROPÄISCHE PATENTSCHRIFT

(45) Veröffentlichungstag der Patentschrift : **27.02.91 Patentblatt 91/09**

(51) Int. Cl.$^5$ : **C07C 381/12,** C07F 7/08, C08F 4/00, G03F 7/26

(21) Anmeldenummer : **88110061.4**

(22) Anmeldetag : **24.06.88**

(54) **Sulfoniumsalze mit säurelabilen Gruppierungen.**

(30) Priorität : **01.07.87 DE 3721740**

(43) Veröffentlichungstag der Anmeldung : **04.01.89 Patentblatt 89/01**

(45) Bekanntmachung des Hinweises auf die Patenterteilung : **27.02.91 Patentblatt 91/09**

(84) Benannte Vertragsstaaten : **AT BE CH DE FR GB IT LI NL SE**

(56) Entgegenhaltungen : **DE-A- 2 004 814**

(73) Patentinhaber : **BASF Aktiengesellschaft**
**Carl-Bosch-Strasse 38**
**D-6700 Ludwigshafen (DE)**

(72) Erfinder : **Schwalm, Reinhold, Dr.**
**Am Huettenwingert 53**
**D-6706 Wachenheim (DE)**
Erfinder : **Boettcher, Andreas, Dr.**
**Konrad-Adenauer-Ring 38**
**D-6907 Nussloch (DE)**

**EP 0 297 442 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue Saint-Denis, 75001 PARIS

**Beschreibung**

Die Erfindung betrifft neue Sulfoniumsalze, die zusätzlich zur Sulfoniumgruppierung säurelabile Gruppen enthalten. Diese Verbindungen erzeugen unter der Einwirkung von Strahlung eine Säure, die die säurelabilen Gruppen abspaltet und damit das Löslichkeitsverhalten der Verbindungen drastisch ändert. Die Verbindungen eignen sich besonders als Fotoinitiatoren für die kationische Polymerisation, sowie als Initiatoren in Fotoresisten.

Sulfoniumsalze sind seit langem in der Literatur bekannt, z.B. H.M. Pitt US 2,807,648 (1957) ; W. Hahn u. R. Stroh US 2,833,827 (1958) ; G.H. Wiegand and W.E. McEwen, J. Org. Chem., 33, 2671 (1968).

Als Fotoinitiatoren kommen fast ausschließlich Sulfoniumsalze mit komplexen, nichtnucleophilen Gegenionen in Frage, wie die von Crivello entwickelten Fotoinitiatoren für die kationische Polymerisation (z.B. US 4,058,400 und US 4,058,401). Einen Überblick über die Verwendung von Oniumsalzen bei der kationischen Polymerisation gibt Crivello in "Cationic Polymerisation - Iodonium and Sulfonium Salt Photoinitiators, Advances in polym. Sci., 62, 1-48 (1984)".

Die Verwendung von Oniumsalzen in Fotoresistmaterialien ist z.B. in "Possibilities for Photoimaging Using Onium Salts, Crivello in Corporate Research and Development, General Electric, Schenectady, New York (1983)", sowie von Ito and Willson in Org. Ctgs. and App. Polym. Sci. Proc. 48, 60 (1983) and US 4,491,628 beschrieben.

Die bisher beschriebenen Sulfoniumsalze sind sehr effektive Initiatoren für die Polymerisation bzw. effektive Säurespender in Fotoresistmaterialien ; keines der bekannten Sulfoniumsalze enthält jedoch säurelabile Gruppen, die durch Einwirkung von Strahlung abgespalten werden können, und das Löslichkeitsverhalten der Verbindungen drastisch ändern.

Aufgabe der vorliegenden Erfindung war es, Fotoinitiatoren zur Verfügung zu stellen, die eine hohe Sensitivität aufweisen, möglichst in einem breiten Bereich des Spektrums der elektromagnetischen Wellen effektiv sind und ihr Löslichkeitsverhalten durch die Belichtung drastisch ändern.

Überraschenderweise wurde gefunden, daß man spezielle Sulfoniumsalze herstellen kann, die neben der Sulfoniumgruppe zusätzlich im gleichen Molekül säurelabile Gruppen enthalten, die die gestellte Aufgabe hervorragend lösen.

Gegenstand der vorliegenden Erfindung sind Sulfoniumsalze der allgemeinen Formel (I)

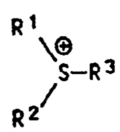 $X^{\ominus}$ (I)

worin $R^1$, $R^2$ und $R^3$ untereinander gleich oder verschieden sind und für aliphatische und/oder aromatische Reste, die gegebenenfalls Heteroatome enthalten, stehen oder zwei der Reste $R^1$ bis $R^3$ miteinander zu einem Ring verknüpft sind, mit der Maßgabe, daß mindestens einer der Reste $R^1$ bis $R^3$ mindestens eine durch Säure spaltbare Gruppierung enthält, wobei einer der Reste $R^1$ bis $R^3$ mit einem oder mehreren weiteren Sulfoniumsalzresten, gegebenenfalls über durch Säure spaltbare Gruppierungen, verknüpft sein kann, und $X^{\ominus}$ ein nichtnukleophiles Gegenion bedeutet.

Bevorzugt enthalten die Sulfoniumsalze als durch Säure spaltbare Gruppierungen tert. -Butoxicarbonylgruppen und/oder Trialkylsilylgruppen.

Die erfindungsgemäßen Sulfoniumsalze können pro Molekül eine oder mehrere, wie z.B. zwei oder drei, durch Säure spaltbare Gruppierungen enthalten.

Die erfindungsgemäßen Verbindungen sind sensitiv gegenüber Kurzwelligen UV-, Elektronen- und Röntgenstrahlen.

Gegenstand der vorliegenden Erfindung ist auch ein Verfahren zur Herstellung der erfindungsgemäßen Sulfoniumsalze, wobei Hydroxyphenylsulfoniumsalze mit nichtnucleophilen Gegenionen an der phenolischen Funktion auf übliche Weise verestert oder verethert werden.

Als Gegenionen eignen sich besonders komplexe nichtnucleophile Metallhalogenide.

Die Sulfoniumsalze mit komplexen Gegenionen erzeugen bei Einwirkung von Strahlung eine starke Säure, die die säurelabile Gruppierung abspaltet und die alkaliunlöslichen Substanzen in basenlösliche phenolische Verbindungen umwandelt.

Da die Abspaltungsreaktionen katalytisch ist, wird die Säure nicht verbraucht, sondern kann weitere Reaktionen, wie die Polymerisation von kationisch polymerisierbaren Monomeren bzw. die Abspaltung von säurelabilen Gruppen, wie z.B. in Polyacetalen oder Poly-t-butylmethacrylat, initiieren. Die erfindungsgemäßen Sulfoniumsalze ändern ihr Löslichkeitsverhalten dahin, daß sie anschließend wäßrig-alkalisch ausgewaschen werden können.

In der allgemeinen Formel (I) für die erfindungsgemäßen Sulfoniumsalze

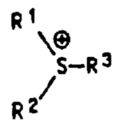 $X^{\ominus}$ (I)

können $R^1$, $R^2$ und $R^3$ untereinander gleich oder verschieden sein und für aliphatische Reste, wie z.B. Alkylgruppen mit 1 bis 12, vorzugsweise 1 bis 6 Kohlenstoffatomen, wie z.B. Methyl-, Ethyl-, n-Propyl-, i-Propyl-, n-Butyl-, i-Butyl-, tert. -Butyl-, Pentyl- oder Hexylgruppen, für cycloaliphatische Reste, wie z.B. Cyclohexyl- oder Cyclopentylgruppen, die gegebenenfalls jeweils substituiert sein können, für Arylreste, wie z.B. Phenyl- oder Naphthylreste, durch 1 bis 4 Alkylgruppen mit 1 bis 6, vorzugsweise 1 bis 4 Kohlenstoffatomen, Alkoxygruppen mit 1 bis 4, vorzugsweise 1 bis 3 Kohlenstoffatomen, ein oder zwei Halogenatome, wie z.B. Fluor, Chlor oder Brom substituierte Arylreste, wie z.B. Methyl-phenyl-, Methoxy-phenyl-, Chlorophenyl-, Brom-phenyl-, Dichlorphenyl- oder Dimethyl-phenylreste stehen oder zwei der Reste $R^1$ bis $R^3$ können miteinander zu einem Ring, insbesondere einem Fünf- oder Sechsring verbunden sein, wobei mindestens einer der Reste $R^1$ bis $R^3$ mindestens eine durch Säure spaltbare Gruppierung enthält.

Beispiele sind Sulfoniumsalze der allgemeinen Formel (I) in denen
$R^3$ z.B. für 4-t-Butoxicarbonyloxi-phenyl-, 4-t-Butoxicarbonyloxi-3,5-dimethyl-phenyl-, 4-t-Butoxicarbonyloxi-3-methyl-phenyl-, 4-t-Butoxicarbonyloxi-2-methyl-phenyl-, 4-t-Butoxicarbonyloxi-3,5-dimethoxi-phenyl-, 4-t-Butoxicarbonyloxi-3,5-diphenyl-phenyl-, 4-t-Butoxicarbonyloxi-1-naphthyl-, 4-Trimethylsilyl-oxi- phenyl-, 4-Trimethylsilyl-oxi-1-naphthyl- stehen oder auch solche, in denen $R^1$ und $R^2$ beispielsweise zu Tetramethylengruppen verbrückt sind und $R^3$ die gleiche Bedeutung wie vorstehend hat :

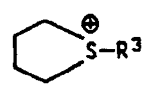

oder Verbindungen, in denen $R^1$ für Methyl und $R^2$ für Phenyl bzw. Tolyl stehen und $R^3$ für ein substituiertes Phenylderivat mit säurespaltbaren Gruppen steht, wie z.B.

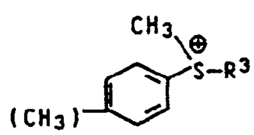

worin $R^3$ z.B. für 4-t-Butoxicarbonyloxi-phenyl-, 2,4-Di-t-butoxicarbonyloxiphenyl-, 4-t-Butoxicarbonyloxi-2-methoxi-phenyl-, 4-Trimethylsilyl-phenyl steht,
oder worin $R^1$ für Phenyl oder $C_1$- bis $C_{12}$-substituiertes Phenyl oder halogensubstituiertes Phenyl steht und $R^2$ und $R^3$ substituierte Phenylderivate mit säurespaltbaren Gruppen sind, wie z.B.

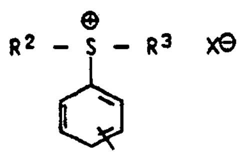

worin $R^2$ und $R^3$ z.B. 4-t-Butoxicarbonyloxi-phenyl-, 4-Trimethylsilyloxiphenyl-, 4-t-Butyl-dimethyl-silyloxiphenyl-, 4-t-Butoxicarbonyloxi-3,5-dimethyl-phenyl- oder $R^1$, $R^2$ und $R^3$ untereinander gleich sind, d.h. Sulfoniumsalze, in denen diese Reste mit säurespaltbaren Gruppen dreimal enthalten sind.

Weiterhin kommen auch Verbindungen der allgemeinen Formel (I) in Betracht, worin einer der Reste

$R^1$ bis $R^3$ mit einem oder mehreren weiteren Sulfoniumsalzresten, gegebenenfalls über durch Säure spaltbare Gruppierungen verknüpft ist, die also ebenfalls mehrere Sulfoniumgruppen im Molekül aufweisen wie, z.B.

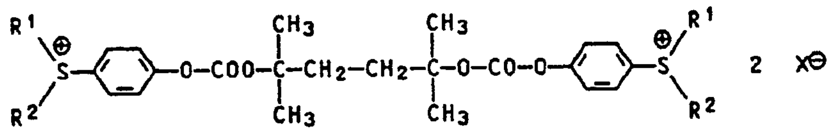

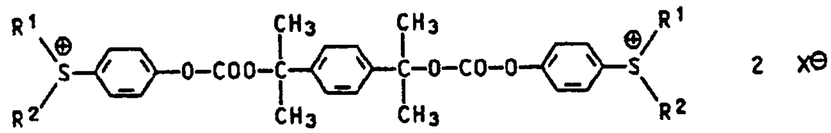

Weitere Beispiele für erfindungsgemäße Sulfoniumsalze sind nachfolgend aufgeführt :

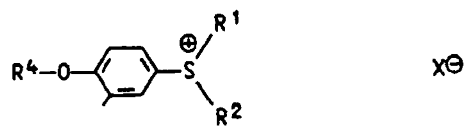

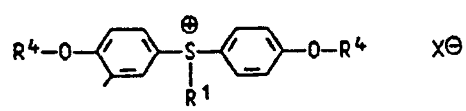

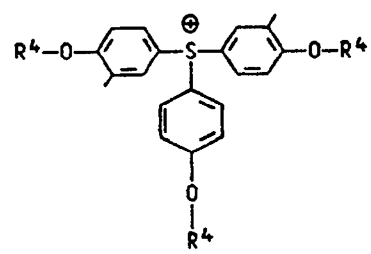

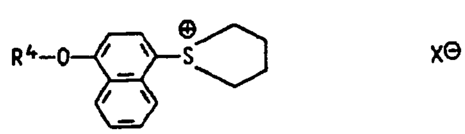

worin $X^{\ominus}$ Halogen, bevorzugt jedoch komplexe Anionen wie $BF_4^{\ominus}$, $AsF_6^{\ominus}$, $SbF_6^{\ominus}$, $PF_6^{\ominus}$ bedeuten kann ;
$R^1$ und $R^2$ wie oben angegeben, Alkyl-, Aryl- oder subst. Arylgruppen bedeuten oder miteinander zu einem Ring verknüpft sind (d.h. bivalent z.B.tetramethylen) ;
$R^4$ bedeutet t-Butoxicarbonyl- oder Trialkylsilylreste, wie z.B. Trimethylsilyl-, t-Butyl-dimethylsilylgruppen.

Zur Herstellung der erfindungsgemäßen Sulfoniumsalze ist folgendes auszuführen :

Die erfindungsgemäßen Sulfoniumsalze können nach üblichen Methoden der organischen Chemie zur Synthese von Estern, Carbonaten und Ethern hergestellt werden, indem man von bekannten Sulfoniumsalzen mit phenolischen Gruppen ausgeht und diese so umsetzt, daß t-Butylester, t-Butylcarbonate oder Silylether von Phenolen entstehen.

Erfindungsgemäß wird ein Verfahren zur Synthese der Verbindungen mit säurelabilen Carbonat bzw. Estergruppierungen vorgeschlagen, bei dem man Hydroxiphenyl-sulfoniumsalze mit nichtnucleophilen

Gegenionen mit Basen behandelt und anschließend mit aktivierten Carbonylverbindungen umsetzt. Die säurelabilen Silylgruppierungen führt man ein, indem man die genannten Hydroxiphenyl-sulfoniumsalze mit den gängigen Silylierungsreagentien, wie Hexamethyl-disilazan oder Silylchloriden umsetzt.

Hydroxiphenyl-dialkyl-sulfoniumsalze, die bereits ein nichtnucleophiles Gegenionen enthalten, können beispielsweise nach einer Synthesevorschrift in J. Polym. Sci., Chem. Ed., 18, 1021 (1980) hergestellt werden. Diese Sulfoniumsalze versetzt man nach dem erfindungsgemäßen Verfahren vorzugsweise mit Kalium-t-butylat in trockenem Tetrahydrofuran und tropft anschließend eine Lösung aus Di-t-butyldicarbonat in Tetrahydrofuran (=THF) zu. Nach der Aufarbeitung und Umkristallisation erhält man die reinen Sulfoniumsalze.

Alternativ kann man die Hydroxyphenyl-dialkyl-sulfoniumsalze mit aktivierten Carbonylverbindungen, wie z.B. t-Butyloxicarbonyl-N-imidazol, umsetzen.

Bis-(hydroxiphenyl)-aryl-sulfoniumsalze kan man beispielsweise nach der Methode von Crivello in J. Polym. Sci., Chem. Ed. 18, 2697 (1980) herstellen, indem man Diaryliodoniumsalze mit nichtnucleophilen Gegenionen mit z.B. Bis-(hydroxyphenyl)-sulfiden unter Kupfer (II) -Katalyse umsetzt. Die so hergestellten Sulfoniumsalze lassen sich ebenfalls in die Derivate mit Carbonat, Ester oder Ethergruppen überführen.

Tris-(hydroxyphenyl-) sulfoniumsalze lassen sich beispielsweise nach einer Synthesevorschrift aus US 2,833,827 (1958) herstellen und analog dem angegebenen erfindungsgemäßen Verfahren derivatisieren.

Am Beispiel des erfindungsgemäßen Sulfoniumsalzes, Phenyl-bis-(t-butyloxicarbonyloxid-phenyl-) sulfonium hexafluoroarsenat wird gezeigt, daß die hydrophobe, alkaliunlösliche Ausgangsverbindung durch Belichtung und einen anschließenden Ausheizschritt in das phenolische Derivat umgewandelt werden kann.

Die Verwendung der Sulfoniumsalze als Fotoinitiatoren für die Abspaltung von säurelabilen Seitengruppen in Fotoresistmaterialien läßt sich zeigen, indem eine Fotoresistlösung aus Poly-(t-butyl-methacrylat) und 20 Gew.%, bezogen auf das Polymere, eines erfindungsgemäßen Sulfoniumsalzes, auf einen Siliziumwafer aufgeschleudert, bildmäßig bestrahlt, ausgeheizt und entwickelt wird. Die belichteten Bereiche lassen sich mit einem alkalischen Entwickler vollständig abtragen, während in den unbelichteten Bereichen kein Abtrag stattfindet. Bei Einsatz der üblicherweise verwendeten Triarylsulfoniumsalze bleibt eine dünne Restschicht zurück bzw. ist zum sauberen Entwickeln ein Cosolvens zum Lösen der Sulfoniumsalze notwendig.

Die in den Beispielen genannten Teile und Prozente sind, soweit nicht anders angegeben, Gewichtsteile bzw. Gewichtsprozente.

Beispiel 1 :

Herstellung von Dimethyl-4-t-butoxicarbonyloxi-phenyl-sulfoniumhexafluoroarsenat

Dimethyl-4-hydroxiphenyl-sulfoniumhexafluoroarsenat wird gemäß der Synthesevorschrift in J. Polym. Sci., Polym. Chem. Ed., 18, 1021 (1980), hergestellt. Aus Phenol und Dimethylsulfoxid in Methanol unter Durchleitung von trockenem HCl wird zuerst das Sulfoniumchlorid erhalten, welches in einer anschließenden Metathesereaktion mit Kaliumhexafluoroarsenat in Dimethyl-4-hydroxiphenyl-sulfoniumhexafluoroarsenat überführt wird.

2,0 Teile dieses Salzes werden in 55 Teilen trockenem Tetrahydrofuran unter $N_2$-Durchleitung gelöst. Dann wird 1 Teil Kalium-t-butylat zugegeben und anschließend 10 Minuten gerührt. Dazu tropft man eine Lösung von 1,27 Teilen Di-t-butyl-dicarbonat in 10 Teilen Tetrahydrofuran und rührt eine Stunde. Die Reaktionsmischung wird in 50 Teile Eiswasser gegeben und mit Ethylacetat mehrmals extrahiert. Die kombinierten Ethylacetatfraktionen werden über Magnesiumsulfat getrocknet und Ethylacetat wird anschließend abgezogen. Das so erhaltene Rohprodukt wird zweimal aus Ethanol umkristallisiert. Man erhält das reine Dimethyl-4-t-butoxicarbonyloxi-phenyl-sulfoniumhexafluoroarsenat in einer Ausbeute von 1,5 Teilen.

NMR 1,5 ppm (S, 9H); 3,3 ppm (S, 6H); 7,65 ppm und 8,15 ppm (para-subst. Arom. je D, 4H)

IR Ar-O-CO-O-aliph. 1760 cm -1

| Elementaranalyse | | C | H | S | As | F |
|---|---|---|---|---|---|---|
| | gef. | 35,0 | 4,3 | 7,6 | 16,7 | 25,4 |
| | ber. | 35,1 | 4,3 | 7,2 | 16,9 | 25,7 |

Das Dimethyl-4-t-butoxicarbonyloxiphenyl-sulfonium-hexafluoroarsenat erhält man auch durch Umsetzung des Dimethyl-4-hydroxyphenyl-sulfoniumhexafluoroarsenats (3,3 Teile) mit Imidazol-N-carbonsäure-t-butylester(1,9 Teile) in 15 Teilen Tetrahydrofuran. Das Reaktionsgemisch wird 8 Stunden auf 70°C erwärmt und nach dem Abkühlen wird das Tetrahydrofuran abdestilliert und der Rückstand aus Ethanol umkristallisiert.

Analog können auch andere Salze, wie Hexafluoroantimonat und Hexafluorophosphat hergestellt werden.

Beispiel 2 :

Synthese von Phenyl-bis-(4-t-butoxicarbonyloxiphenyl-)-sulfoniumhexafluoroarsenat

In einem 100 ml-Zweihalskolben mit Rückflußkühler und Magnetrührer werden unter $N_2$-Durchleitung 11,75 g (0,025 mol) Diphenyliodoniumhexafluoroarsenat, 5,46 g (0,025 mol) 4,4'-Dihydroxi-diphenylsulfid und 0,2 g Kupfer (II) -acetat vorgelegt. Die Mischung wird unter $N_2$ 3 Stunden auf 125°C erhitzt, anschließend in ein Becherglas gegeben und mehrere Male mit Diethylether extrahiert. Das Rohprodukt wird aus Chloroform/Diethylether umkristallisiert. Ausbeute : 6,3 g NMR- und IR-Spektren zeigen, daß das neu hergestellte Produkt Phenyl-bis-(4-hydroxiphenyl-)-sulfoniumhexafluoroarsenat ist.

6,3 g des synthetisierten Phenyl-bis-(4-hydroxiphenyl-)-sulfoniumhexafluoroarsenates werden in 100 ml trockenem Tetrahydrofuran unter $N_2$-Durchleitung gelöst. Dann gibt man 2,9 g Kalium-t-butylat zu und rührt anschließend nach 10 Minuten nach. Dazu tropft man 6,24 g Di-t-butyl-dicarbonat in 20 ml THF und rührt eine weitere Stunde nach. Die Reaktionsmischung wird in 150 g Eiswasser gegeben und mit Ethylacetat mehrmals extrahiert. Die kombinierten Ethylacetatfraktionen werden über Magnesiumsulfat getrocknet und das Lösungsmittel wird abgezogen. Nach dem Umkristallisieren erhält man 7,0 g reines Phenyl-bis-(4-t-butoxicarbonyloxiphenyl-)-sulfoniumhexafluoroarsenat

NMR 1,5 ppm (S, 18H) ; 7,5 ppm (D, 4H) ; 7,7 ppm (M, 5H) ; 7,8 ppm (D, 4H)
IR (C=O, Carbonat) 1760 $cm^{-1}$
Schmelzpunkt : 128°C

Beispiel 3 :

Phenyl-bis-(4-t-butoxicarbonyloxi-phenyl-)-sulfoniumhexafluoroarsenat wird auf eine Natriumchloridplatte aufgebracht und 30 Sekunden bei 120°C ausgeheizt. Das IR-Spektrum zeigt eine scharfe Carbonylbande bei 1760 $cm^{-1}$ (Carbonat) und kein phenolisches OH ; es ist keine Veränderung gegenüber dem nicht ausgeheizten Spektrum zu sehen. Belichtet man nun 10 Sekunden mit Excimer-Laserlicht der Wellenlänge 248 nm und heizt anschließend 30 Sekunden bei 120°C nach, so ist die Carbonylbande vollständig verschwunden und dafür eine OH-Bande bei 3500 $cm^{-1}$ entstanden.

Das Ausgangsprodukt ist in verdünnter Natriumcarbonatlösung unlöslich, während ein gemäß dem vorstehenden Beispiel behandeltes Produkt vollständig in diesem alkalischen Lösungsmittel gelöst wird.

Beispiel 4 :

Synthese von 4-(1-trimethylsilyloxynaphthyl)-tetrahydro-thiopheniumchlorid

5,33 g (20 mol) 4-(1-hydroxynaphthyl)tetrahydrothiopheniumchlorid werden vorgelegt und 2,4 ml Hexamethyl-disilazan bei 25°C während 45 Minuten zugetropft. Es werden weitere 5 ml Hexamethyl-disilazan zugegeben und 7 Stunden bei 100°C reagieren lassen. Von der entstrehenden gelben Lösung wird das überschüssige Hexamethyl-disilazan im Ölpumpenvakuum abdestilliert. Est bleiben 6,5 g eines gelben Öles zurück. Das NMR-Spektrum entspricht dem erwarteten silylierten Produkt.

Beispiel 5 :

Eine Mischung aus 30 Teilen destilliertem Styrol, 0,5 Teilen des nach Beispiel 2 hergestellten Phenyl-bis-(t-butyloxicarbonyloxiphenyl-)-sulfoniumhexafluoroarsenats und 10 Teilen Tetrachlormethan wird unter Stickstoff 15 Minuten mit einer Cd-Xe-Lampe belichtet. Es findet eine exotherme Polymerisation statt und die Reaktionslösung wird viskos. Danach wird noch für 30 Minuten bei ca. 80°C erhitzt, die Lösung verdünnt, mit verdünnter Natriumcarbonatlösung ausgeschüttelt und in Methanol gefällt. Das IR-Spektrum zeigt, daß es sich um reines Polystyrol handelt und keine phenolische Absorption des zerfallenen Initiators vorhanden

ist.

Beispiel 6 :

Handelsübliches Poly-(t-butylmethacrylat) ($\overline{M}_n$ 83.000) wird in Methylglykolacetat gelöst und mit 20%, bezogen auf Poly-(t-butylmethacrylat), Dimethyl-4-t-butoxicarbonyloxi-phenylsulfonium-hexafluorarsenat vermischt,so daß eine Lösung von insgesamt 25% Feststoffgehalt erhalten wird. Diese Lösung wird durch ein Filter mit einem Porendurchmesser von 0,2 µm filtriert und mit 3000 Umdrehungen/min auf einen Siliziumwafer aufgeschleudert. Der Film wird 5 Minuten bei 90°C ausgeheizt, bildmäßig durch eine Quarzmaske belichtet und anschließend 1 Minute bei 120°C ausgeheizt. Mit einem alkalischen Entwickler von pH-Wert 13,0 lassen sich die belichteten Bereiche vollständig entwickeln.

Bei einem Vergleichsversuch mit Triphenylsulfoniumhexafluoroarsenat anstatt des erfindungsgemäßen Sulfoniumsalzes blieb eine Restschicht von ca. 0,1 µm auf dem Wafer in den belichteten Bereichen zurück.

## Ansprüche

1. Sulfoniumsalze der allgemeinen Formel (I)

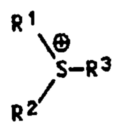 $X^{\ominus}$ (I)

worin $R^1$, $R^2$ und $R^3$ untereinander gleich oder verschieden sind und für aliphatische und/oder aromatische Reste, die gegebenenfalls Heteroatome enthalten, stehen oder zwei der Reste $R^1$ bis $R^3$ miteinander zu einem Ring verknüpft sind. mit der Maßgabe, daß mindestens einer der Reste $R^1$ bis $R^3$ mindestens eine durch Säure spaltbare Gruppierung enthält, wobei einer der Reste $R^1$ bis $R^3$ mit einem oder mehreren weiteren Sulfoniumsalzresten, gegebenenfalls über durch Säure spaltbare Gruppierungen, verknüpft sein kann, und $X^{\ominus}$ ein nichtnukleophiles Gegenion bedeutet.

2. Sulfoniumsalze nach Anspruch 1, dadurch gekennzeichnet, daß die durch Säure spaltbare Gruppierung eine tert. -Butoxicarbonylgruppe ist.

3. Sulfoniumsalze nach Anspruch 1, dadurch gekennzeichnet, daß die durch Säure spaltbare Gruppierung eine Trialkylsilylgruppe ist.

4. Sulfoniumsalze nach einem der vorhergehenden Ansprüche, dadurch gekennzeichnet, daß sie pro Molekül eine durch Säure spaltbare Gruppierung enthalten.

5. Sulfoniumsalze nach einem der Ansprüche 1 bis 3, dadurch gekennzeichnet, daß sie pro Molekül zwei durch Säure spaltbare Gruppierungen enthalten.

6. Sulfoniumsalze nach einem der Ansprüche 1 bis 3, dadurch gekennzeichnet, daß sie pro Molekül drei durch Säure spaltbare Gruppierungen enthalten.

7. Verfahren zur Herstellung der Sulfoniumsalze nach einem der Ansprüche 1 bis 6, dadurch gekennzeichnet, daß Hydroxyphenylsulfoniumsalze mit nichtnucleophilen Gegenionen an der phenolischen Funktion auf übliche Weise verestert oder verethert werden.

8. Verwendung der Sulfoniumsalze nach einem der Ansprüche 1 bis 6, als Fotoinitiatoren für kationische Polymerisationen und in lichtempfindlichen Beschichtungsmaterialien.

## Claims

1. A sulfonium salt of the formula (I)

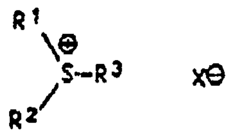

(I)

where $R^1$, $R^2$ and $R^3$ are identical or different and are each an aliphatic or aromatic radical which may contain heteroatoms, or two of the radicals $R^1$ and $R^3$ are bonded to one another to form a ring, with the proviso that one or more of the radicals $R^1$ to $R^3$ contain one or more acid-cleavable groups, and one of the radicals $R^1$ to $R^3$ can be bonded to one or more further sulfonium salt radicals, if desired via acid-cleavable groups, and $X^{\ominus}$ is a non-nucleophilic counter-ion.

2. A sulfonium salt as claimed in claim 1, wherein the acid-cleavable group is tert-butoxycarbonyl.

3. A sulfonium salt as claimed in claim 1, wherein the acid-cleavable group is trialkylsilyl.

4. A sulfonium salt as claimed in any of the preceding claims, which contains one acid-cleavable group per molecule.

5. A sulfonium salt as claimed in any of claims 1 to 3, which contains two acid-cleavable groups per molecule.

6. A sulfonium salt as claimed in any of claims 1 to O.A. 0050/39289 3, which contains three acid-cleavable groups per molecule.

7. A process for the preparation of a sulfonium salt as claimed in any of claims 1 to 6, wherein a hydroxyphenylsulfonium salt having a non-nucleophilic counterion is esterified or etherified at the phenolic function in a conventional manner.

8. The use of a sulfonium salt as claimed in any of the claims 1 to 6 as a photoinitiator for cationic polymerization and in light-sensitive coating materials.

**Revendications**

1. Sels de sulfonium de formule générale I

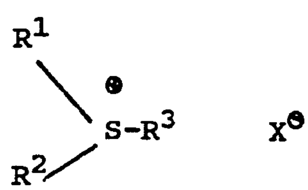

(I)

dans laquelle $R^1$, $R^2$ et $R^3$, ayant des significations identiques ou différentes, représentent chacun un radical aliphatique ou aromatique qui peut contenir le cas échéant des hétéroatomes, ou bien deux des substituants $R^1$ à $R^3$ sont reliés entre eux avec formation d'un cycle, sous réserve que l'un au moins des substituants $R^1$ à $R^3$ contient au moins un groupement scindable par un acide, l'un des substituants $R^1$ à $R^3$ pouvant être relié avec un ou plusieurs autres radicaux de sels de sulfonium, éventuellement par l'intermédiaire de groupements scindables par un acide, et $X^{\ominus}$ représente un ion complémentaire non nucléophile.

2. Sels de sulfonium selon la revendication 1, caractérisés en ce que le groupement scindable par un acide est un groupe tert-butoxycarbonyle.

3. Sels de sulfonium selon la revendication 1, caractérisés en ce que le groupement scindable par un acide est un groupe trialkylsilyle.

4. Sels de sulfonium selon une des revendications qui précèdent, caractérisés en ce qu'ils contiennent un groupement scindable par un acide, par molécule.

5. Sels de sulfonium selon une des revendications 1 à 3, caractérisés en ce qu'ils contiennent deux groupements scindables par un acide, par molécule.

6. Sels de sulfonium selon une des revendications 1 à 3, caractérisés en ce qu'ils contiennent trois groupements scindables par un acide, par molécule.

7. Procédé de préparation des sels de sulfonium selon une des revendications 1 à 6, caractérisé en ce que, partant de sels d'hydroxyphénylsulfonium dont les ions complémentaires sont non nucléophiles,

on les éthérifie ou estérifie de la manière habituelle sur la fonction phénolique.

8. Utilisation des sels de sulfonium selon une des revendications 1 à 6, en tant que photo-inducteurs pour les polymérisations cationiques et dans les matériaux de revêtement photosensibles.